# EUROPÄISCHE PATENTSCHRIFT

(11) **EP 0 985 665 B1**
(45) Veröffentlichungstag und Bekanntmachung des Hinweises auf die Patenterteilung: **11.12.2002**
(21) Anmeldenummer: 99116580.4
(22) Anmeldetag: 25.08.1999
(51) Int. Cl.: C07D 207/26, C07C 381/12, C07C 321/14

(54) **Verfahren zur Herstellung von 3-Amino-2-oxo-pyrrolidinen, Zwischenprodukte und deren Verwendung**
Process for the preparation of 3-amino-2-oxo-pyrrolidines, intermediates and use thereof
Procédé de préparation de 3-amino-2-oxo-pyrrolidines, intermédiaires et leur utilisation

(30) Priorität: 11.09.1998 DE 19841895
(43) Veröffentlichungstag der Anmeldung: 15.03.2000
(73) Patentinhaber: Degussa AG, 40474 Düsseldorf (DE)
(72) Erfinder: Drauz, Karlheinz, Prof., 63579 Freigericht (DE); Klement, Ingo, Dr., 35415 Pohlheim-Garbenteich (DE); Knaup, Günter, Dr., 63486 Bruchköbel (DE)

(56) Entgegenhaltungen:
- EP-A- 0 026 640
- US-A- 5 576 447
- R.M. FREIDINGER ET AL.: JOURNAL OF ORGANIC CHEMISTRY, Bd. 47, - 1982 Seiten 104-109, XP002122929 EASTON US
- J E SEMPLE: "Design, synthesis, and evolution of a novel, selective, and orally bioavailable class of thrombin inhibitors: P1-argininal derivatives incorporating P3-4 lactam sulfonamide moieties" JOURNAL OF MEDICINAL CHEMISTRY,US,AMERICAN CHEMICAL SOCIETY. WASHINGTON, Bd. 39, Nr. 23, 1996, Seite 4531-4536 XP002081550 ISSN: 0022-2623
- R.J. CREGGE ET AL.: JOURNAL OF MEDICINAL CHEMISTRY, Bd. 41, Juli 1998 (1998-07), Seiten 2461-2480, XP002122930 WASHINGTON US
- TOENNIES ET AL: 'Methionine Studies. VII. Sulfonium Derivatives' J. AM. CHEM. SOC. Bd. 67, Mai 1945, Seiten 849 - 851
- GUNDLACH ET AL: 'The Reaction of Iodoacetate with Methionine' J. BIOL. CHEM. Bd. 234, Nr. 7, Juli 1959, Seiten 1761 - 1764
- NATELSON ET AL: 'Preparation of D-, DL- and L-Homoserine Lactone from Methionine' MICROCHEMICAL JOURNAL Bd. 40, Nr. 2, Oktober 1989, Seiten 226 - 232
- GRAF ET AL: 'Decrease of alpha-Helix Potential and Biological Activity of beta-Endorphin in Response to Modifications of MET' NEUROPEPTIDES Bd. 1, Nr. 1, Juli 1980, Seiten 47 - 51

## Beschreibung

Die vorliegende Erfindung betrifft ein Verfahren zur Herstellung von Verbindungen der allgemeinen Formel I und deren Salze 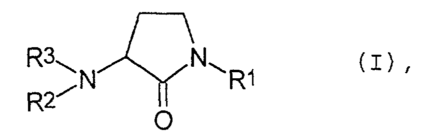 worin:
R¹ bedeutet H, (C₁-C₈)-Alkyl, (C₂-C₈)-Alkenyl, (C₂-C₈)-Alkinyl, (C₂-C₈)-Alkoxyalkyl, (C₁-C₈)-Acyl, welche gegebenenfalls linear oder verzweigt vorliegen können sowie einfach oder mehrfach mit Halogenen, N-, O-, P-, S-atomhaltigen Resten substituiert sein können, (C₃-C₇)-Cycloalkyl, das gesättigt oder ungesättigt sowie einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein kann oder im Ring Heteroatome wie N, O, P, S enthalten kann, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl,
   wobei die genannten Cyclen gegebenenfalls einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, mit Nitril, mit C(O)NH₂ oder N-, O-, P-, S-atomhaltigen Resten substituiert sein können, N-verknüpfter Aminosäure- oder Peptidrest,
R² bedeutet H, (C₁-C₈)-Alkyl, (C₂-C₈)-Alkenyl, (C₂-C₈)-Alkinyl, (C₂-C₈)-Alkoxyalkyl, welche gegebenenfalls linear oder verzweigt vorliegen können sowie einfach oder mehrfach mit Halogenen, N-, O-, P-, S-atomhaltigen Resten substituiert sein können, (C₃-C₇)-Cycloalkyl, das gesättigt oder ungesättigt sowie einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈) -Alkoxy, (C₁-C₈)-Alkoxyalkyl, mit Halogenen, N-, O-, P-, S-atomhaltigen Resten substituiert sein kann und/oder im Ring Heteroatome wie N, O, P, S enthalten können, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die genannten Cyclen gegebenenfalls einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen oder N-, O-, P-, S-atomhaltigen Resten substituiert sein können,
R³ bedeutet H, (C₁-C₈)-Acyl, das gegebenenfalls linear oder verzweigt vorliegen kann,
ein C-verknüpfter Aminosäure- oder Peptidrest oder eine gängige Peptidschutzgruppe wie z. B. Formyl, Carbamoyl, Benzyloxycarbonyl, tert.-Butyloxycarbonyl, Allyloxycarbonyl, Trifluoracetyl.

Weiterhin richtet sich die Erfindung auf neue Zwischenprodukte der allgemeinen Formel II 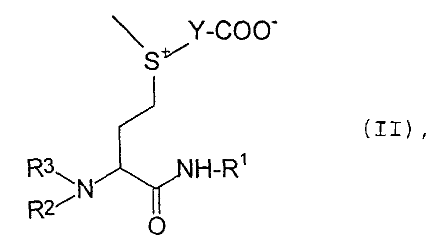 sowie deren Säureadditionssalze, worin R¹, R², R³ die oben angegebene Bedeutung besitzen und Y für einen linearen oder verzweigten (C₁-C₈)-Alkylenrest, der gegebenenfalls mehrfach mit (C₁-C₄)-Alkyl, Halogen, Hydroxy oder Phenyl substituiert sein kann, (C₇-C₁₅)-Arylalkyl, das gegebenenfalls mehrfach mit (C₁-C₄)-Alkyl, Halogen oder Hydroxy substituiert sein kann, Verfahren zu deren Herstellung und deren Verwendung.

Die durch das erfindungsgemäße Verfahren herstellbaren Verbindungen und die neuen Zwischenprodukte sind wertvolle Intermediate zur Produktion von bioaktiven Wirkstoffen. So werden 3-Amino-2-oxo-pyrrolidine bevorzugt als Bausteine für Peptidmimetika eingesetzt, die als Pharmazeutika verwendet werden. In der WO 94/22820 sind beispielsweise am Phenylring substituierte 3-Amino-1-phenyl-2-oxo-pyrroldine als Zwischenprodukte für Thrombozyten Aggregationsinhibitoren beschrieben. Weitere, diese γ-Lactame enthaltende bioaktive Verbindungen sind von Kottirsch et al. (Bioorg. Med. Chem. Lett. 1993, 3, 1675) untersucht worden. In anderen Beispielen werden diese in hochpotenten Neurokinin NK-2 Rezeptor Antagonisten nach Deal et al. (J. Med. Chem. 1992, 35, 4195) eingesetzt.

Die Mehrzahl der bisher angewandten Verfahren zur Herstellung von substituierten 3-Amino-2-oxo-pyrrolidinen besteht darin, die entsprechenden offenkettigen Methioninverbindungen zunächst in deren Dimethylsulfoniumsalze zu überführen und diese mit starken Basen in einem geeigneten Lösungsmittel zu cyclisieren. Von Friedinger et. al (J. Org. Chem. 1982, 47, 104-109) wurde hierfür Methyliodid und Natriumhydrid, was im großtechnischen Prozeß schlecht zu handhaben ist, eingesetzt. In der US 5,484,946 werden für die Alkylierung anstelle des leichtflüchtigen Methyliodids Trimethylsulfonium- bzw. Trimethylsulfoxoniumsalze eingesetzt. Die Cyclisierung wird dann mit Kaliumcarbonat durchgeführt.

Ein wesentlicher Nachteil dieser Verfahren ist die zwangsläufige Freisetzung des extrem geruchsintensiven Dimethylsulfids aus der Methioninvorstufe. Nachteilig erscheint darüber hinaus der erforderliche Einsatz von teuren aprotisch polaren Lösungsmitteln wie z. B. DMSO bei der Cyclisierung mit Kaliumcarbonat.

In der WO 94/22820 wird ein Verfahren erwähnt, bei dem racemische Homoserinderivate, die ausgehend von Butyrolacton hergestellt wurden, mittels Triphenylphosphin und Azodicarbonsäurediestern zu Pyrrolidonen cyclisiert werden. Diese Reagenzien sind jedoch für den Einsatz in einem technischen Verfahren wenig geeignet, da sie relativ teuer sind. Des weiteren liefert diese Variante bei der Cyclisierung eine Reihe von Nebenprodukten, deren Abtrennung vom gewünschten Derivat schwierig und damit zeit- und kostenintensiv ist (K. Nakajima et al. Peptide Chemistry 1983, 77-80).

Aufgabe der vorliegenden Erfindung ist deshalb die Angabe eines weiteren Verfahrens zur Herstellung von γ-Lactamen der allgemeinen Formel I, das die vorgenannten Nachteile des Standes der Technik vermeidet und das vorteilhaft in einem technischen Maßstab durchführbar ist, d. h. möglichst den Einsatz von kritischen Substanzen sowie teuren Reagenzien vermeidet. Unter kritischen Substanzen werden im Sinne der Erfindung solche Verbindungen verstanden, die im großen Maßstab eingesetzt zu besonderen Risiken hinsichtlich der Umwelt oder des Arbeitsschutzes Anlaß geben.

Es ist weiterhin Aufgabe der Erfindung, neue vorteilhaft für die Synthese der γ-Lactame der allgemeinen Formel I einsetzbare Zwischenprodukte anzugeben, Verfahren zu deren Herstellung und eine vorteilhafte Verwendung dieser Zwischenprodukte zu nennen.

Die Lösung dieser und weiterer nicht näher erläuterter Aufgaben, welche sich jedoch ohne weiteres in naheliegender Weise aus dem Stand der Technik ergeben, sind Gegenstand eines Verfahrens mit den Merkmalen des Anspruchs 1. Vorteilhafte Ausgestaltungen des erfindungsgemäßen Verfahrens werden in den von Anspruch 1 abhängigen Unteransprüchen beschrieben. Anspruch 19 löst die Aufgabe zur Angabe von neuen Zwischenprodukten. In Anspruch 22 wird eine vorteilhafte Verwendung der Zwischenprodukte beschreiben.

Dadurch, daß man eine Verbindung der allgemeinen Formel I 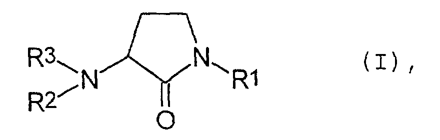 worin:
R¹ bedeutet H, (C₁-C₈)-Alkyl, (C₂-C₈)-Alkenyl, (C₂-C₈)-Alkinyl, (C₂-C₈)-Alkoxyalkyl, (C₁-C₈)-Acyl, welche gegebenenfalls linear oder verzweigt vorliegen können sowie einfach oder mehrfach mit Halogenen, N-, O-, P-, S-atomhaltigen Resten substituiert sein können, (C₃-C₇)-Cycloalkyl, das gesättigt oder ungesättigt sowie einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein kann oder im Ring Heteroatome wie N, O, P, S enthalten kann, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl,
   wobei die genannten Cyclen gegebenenfalls einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, mit Nitril, mit C(O)NH₂ oder N-, O-, P-, S-atomhaltigen Resten substituiert sein können, N-verknüpfter Aminosäure- oder Peptidrest,
R² bedeutet H, (C₁-C₈)-Alkyl, (C₂-C₈)-Alkenyl, (C₂-C₈)-Alkinyl, (C₂-C₈)-Alkoxyalkyl, welche gegebenenfalls linear oder verzweigt vorliegen können sowie einfach oder mehrfach mit Halogenen, N-, O-, P-, S-atomhaltigen Resten substituiert sein können, (C₃-C₇)-Cycloalkyl, das gesättigt oder ungesättigt sowie einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein kann und/oder im Ring Heteroatome wie N, O, P, S enthalten können, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die genannten Cyclen gegebenenfalls einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen oder N-, O-, P-, S-atomhaltigen Resten substituiert sein können,
R³ bedeutet H, (C₁-C₈)-Acyl, das gegebenenfalls linear oder verzweigt vorliegen kann,
   ein C-verknüpfter Aminosäure- oder Peptidrest oder eine gängige Peptidschutzgruppe wie z. B. Formyl, Carbamoyl, Benzyloxycarbonyl, tert.-Butyloxycarbonyl, Allyloxycarbonyl, Trifluoracetyl, durch Cyclisierung von Derivaten der allgemeinen Formel II 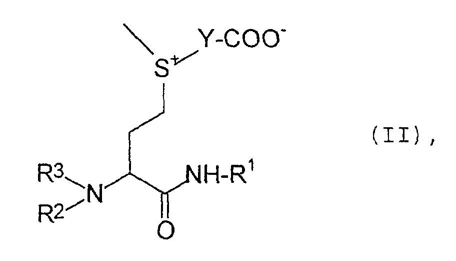 worin R¹, R², R³ die oben angegebene Bedeutung besitzen und Y für einen linearen oder verzweigten (C₁-C₈)-Alkylenrest, der gegebenenfalls mehrfach mit (C₁-C₄)-Alkyl, Halogen, Hydroxy oder Phenyl substituiert sein kann, (C₇-C₁₅)-Arylalkyl, das gegebenenfalls mehrfach mit (C₁-C₄)-Alkyl, Halogen oder Hydroxy substituiert sein kann, synthetisiert, gelangt man in sehr einfacher und dennoch vorteilhafter Weise in guten Ausbeuten zu den gewünschten Verbindungen der allgemeinen Formel I, wobei die für diese Synthese notwendigen relativ preisgünstigen Einsatzstoffe, die einfache Reaktionsführung, die guten Ausbeuten und der Verzicht auf gefährliche oder übelriechende Reagenzien dieses erfindungsgemäße Verfahren für einen großtechnischen Prozeß prädestinieren.

Vorteilhafterweise kann die eben beschriebene Cyclisierung dabei unter basischen Bedingungen erfolgen, wobei als Basen tertiäre Amine wie z. B. Triethylamin, Tributylamin oder anorganische Basen wie Alkali- oder Erdalkalialkoxide, -hydroxide oder -carbonate eingesetzt werden können. Insbesondere geeignet sind Basen wie NaOH, KOH, Ca(OH)₂, Na₂CO₃, K₂CO₃, CaCO₃, CaO, NaHCO₃ oder KHCO₃. Bevorzugt ist der Einsatz von festem Alkalicarbonat, und ganz besonders bevorzugt kann Kaliumcarbonat zur Cyclisierung verwendet werden.

Die Cyclisierung wird bevorzugt in polaren, protischen Lösungsmitteln durchgeführt. Ganz besonders bevorzugt wird Methanol eingesetzt.

Besonders bevorzugt wird das Verfahren mit Verbindungen der allgemeinen Formel II durchgeführt, bei denen Y für -CH₂steht. Ganz besonders bevorzugt ist dieses Verfahren auf Verbindungen der allgemeinen Formel II anzuwenden, bei denen R¹ einen p-Cyanophenyl- oder p-Carbamoylphenylrest darstellt, während R² vorteilhafterweise H und R³ = Benzyloxycarbonyl ist.

Die Cyclisierung kann bei Temperaturen von 0°C bis 150 °C betrieben werden. Bevorzugt werden Temperaturen von 20°C bis 100 °C, ganz besonders bevorzugt Temperaturen von 40°C bis 70 °C bei der Cyclisierung eingestellt.

Die Synthese der Verbindungen der allgemeinen Formel I kann bevorzugt und äußerst einfach aus Verbindungen der allgemeinen Formel II erfolgen, indem II, das aus III (s. u.) herstellbar ist, ohne vorherige Isolierung zu I umgesetzt wird.

Um Derivate des allgemeinen Verbindungstyps I zu erhalten, kann es notwendig sein, weitere Umsetzungen durchzuführen. Diese Umsetzungen betreffen Schutzgruppenabspaltungsreaktionen aus dem zunächst entstandenen Verbindungstyp I und sind dem Fachmann geläufig. Exemplarisch ist eine derartige Umsetzung in Beispiel 4 gezeigt.

Die Verbindungen der allgemeinen Formel II lassen sich vorteilhafterweise aus Derivaten der allgemeinen Formel III 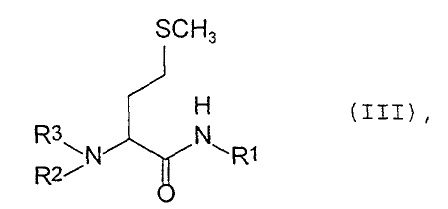 worin R¹, R², R³ die oben angegebene Bedeutung besitzen, durch Umsetzung mit Halogencarbonsäuren der allgemeinen Formel IV

X-Y-COOH (IV)

worin Y die oben angebenene Bedeutung besitzt, X für Halogen steht, bevorzugt in Gegenwart einer Base, herstellen. Geeignet für die Umsetzung mit den Verbindungen des allgemeinen Typs III sind insbesondere folgende Verbindungen des allgemeinen Typs IV:
α-Chloressigsäure, α-Bromessigsäure, 3-Chlor-, Brom- oder Iodpropionsäure, 4-Chlor- oder Brombuttersäure, 2-Bromisobuttersäure, 3-Chlorpivalsäure, α-Bromphenylessigsäure, 2-Bromisovaleriansäure, 2-Brompropionsäure, 2-Brombuttersäure, 2-Bromvaleriansäure, 2-Bromhexansäure, 2-Chlorpropionsäure, 2-Bromoctansäure, 5-Chlor-, Bromvaleriansäure, 6-Bromhexansäure, 3-Chlor-N-buttersäure, 2-Brom-3-methylvaleriansäure, 2-Chlor-N-buttersäure, α-Chlorphenylessigsäure, 2-Chlor-3-phenylpropionsäure, 2-Chlorpentansäure, 2-Chlor-3-methylbuttersäure, 4 -Brommethylbenzoesäure, 4-Chlormethylbenzoesäure, 4-Brommethylphenylessigsäure, 4-(2-Chlorethyl)benzoesäure, p-(β-Brommethyl)benzoesäure, 4-(α-Brommethyl)benzoesäure, 3-Brommethylbenzoesäure, 3-Brombuttersäure, Brompivalsäure, 3-Chlormilchsäure.

Bevorzugt werden α-Halogencarbonsäuren und ganz besonders bevorzugt α-Halogenessigsäuren eingesetzt. Als Halogen wird bevorzugt Chlor oder Brom verwendet. Als Basen können tertiäre Amine wie z. B. Triethylamin, Tributylamin oder anorganische Basen wie Alkali- oder Erdalkalialkoxide, -hydroxide oder -carbonate oder Alkalialkoholate, wie beispielsweise Natriummethylat oder Natriumethylat, eingesetzt werden. Insbesondere geeignet sind Basen wie NaOH, KOH, Ca(OH)₂, Na₂CO₃, K₂CO₃, CaCO₃, CaO, NaHCO₃ oder KHCO₃. Bevorzugt ist der Einsatz von festen Alkalihydroxiden, und ganz besonders bevorzugt kann Kaliumhydroxid verwendet werden. Neben α-Halogencarbonsäuren können auch β-Halogencarbonsäuren verwendet werden.

Prinzipiell können alle Lösungsmittel verwendet werden, in denen die Edukte eine ausreichende Löslichkeit besitzen. Bevorzugt werden jedoch polare protische Lösungsmittel wie z. B. Wasser, Alkohole, Ketone oder Lösungsmittelgemische, die diese enthalten, eingesetzt. Ganz besonders bevorzugt wird die Reaktion in Methanol oder Aceton durchgeführt.

Bei Verwendung von Lösungsmitteln, die mit der eingesetzten Halogencarbonsäure reagieren können (z. B. Bromessigsäure und Wasser zu Glykolsäure), wird die Halogencarbonsäure in einem Überschuß eingesetzt. Bei der Umsetzung mit Bromessigsäure in Methanol und Kaliumhydroxid als Base wurde ein 1,5 facher Überschuß als am geeignetsten gefunden.

Je nach verwendeter Halogencarbonsäure kann die Reaktion zwischen -20°C und 150°C durchgeführt werden. Bei Verwendung der Bromessigsäure wird eine Temperatur von 20 - 100°C, ganz besonders bevorzugt von 40 - 80°C, eingestellt.

Selbstverständlich können anstatt der Halogencarbonsäuren auch Derivate davon, wie beispielsweise Ester der Säuren, zur Reaktion eingesetzt werden. Die Umsetzungen erfolgen in analoger Weise wie oben beschrieben. Vor der anschließenden Cyclisierung muß jedoch eine Verseifung zur entsprechenden Säure stattfinden, die in an sich bekannter Weise durchzuführen ist. Geeignet sind insbesondere die Halogencarbonsäurederivate, in denen in der allgemeinen Formel (IV) das Wasserstoffatom durch (C₁-C₄)-Alkyl, (C₂-C₆)-Alkenyl, (C₂-C₆)-Alkoxyalkyl, Phenyl, das mit (C₁-C₃)-Alkyl oder Halogen substituiert sein kann, Benzyl oder Phenethyl ersetzt ist.

Die Verbindungen der Formel III können nach Literaturverfahren (**Houben-Weyl, Band 15, Teil 2**) aus Verbindungen der allgemeinen Formel V 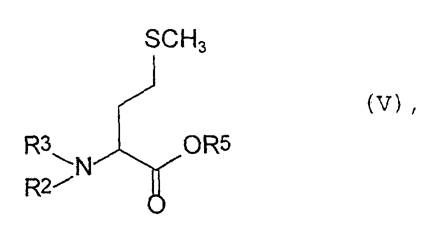 in der R², R³, die oben angegebene Bedeutung besitzen und R⁵ für H steht durch Umsetzung mit Aminen H₂NR¹ hergestellt werden, wobei R¹ die oben angegebene Bedeutung besitzt.

Ein weiterer Aspekt der vorliegenden Erfindung betrifft die vorteilhaften neuen Zwischenprodukte der allgemeinen Formel II 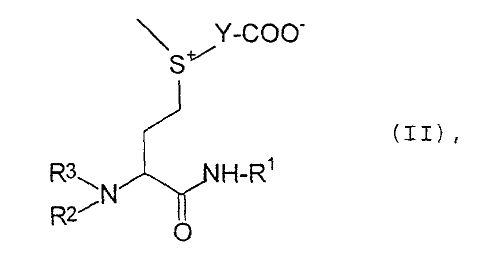 sowie deren Säureadditionssalze, in der R¹,R^{2,} R³ und Y die oben angegebene Bedeutung besitzen. Bevorzugt sind dabei Verbindungen, für die R² Wasserstoff bedeutet, R³ für einen Benzyloxycarbonyl-Rest und Y für -CH₂- steht. Ganz besonders bevorzugt sind Verbindungen bei denen weiterhin R¹ einen p-Cyanophenyl- oder p-Carbamoylphenylrest darstellt.

Die Verbindungen der allgemeinen Formel II werden erfindungsgemäß zur Herstellung von Intermediaten von bioaktiven Wirkstoffen eingesetzt.

Die Darstellung der Verbindungen des allgemeinen Typs II erfolgt wie oben beschrieben aus Derivaten der allgemeinen Formel III mit Halogencarbonsäuren der allgemeinen Formel IV.

Umsetzungen von Methionin bzw. N-Acylmethioninen mit Halogenessigsäuren sind zwar bekannt (G. Toennies, J.L. Kolb, J. Am. Chem. Soc. 67, 849 (1945); H.G. Gundlach, S. Moore, W.H. Stein, J. Biol. Chem. 234, 1761 (1959), die entsprechenden Carboxymethylsulfoniumsalze werden jedoch als instabile Verbindungen beschrieben, die, je nach Reaktionsbedingungen, entweder unter Substitution zu Homoserin- bzw. Homoserinlactonderivaten oder unter Elimination entweder zum Ausgangsprodukt zurück oder zu S-Carboxymethylhomocystein abreagieren. Diese relative Unbeständigkeit wurde für eine einfache Überführung von Methionin mit Bromessigsäure zu Homoserinlacton verwendet (S. Natelson, E.A. Natelson, Microchemical Journal, **40**, 226 - 232 (1989)).

Als einzige Umsetzung eines N-Acyl-methionin-amides mit einer Halogenessigsäure ist die Umsetzung von β-Endorphin im Milligrammmaßstab mit einem 25-fachen Überschuß von Iodessigsäure zum entsprechen S-Carboxymethylmethioninanaloga bekannt (L. Graf et al., Neuropeptides, **1**, 47, 1980).

Es war daher sehr überraschend, daß die Methioninderivate der allgemeinen Formel III durch Umsetzung mit Halogencarbonsäuren einfach und in sehr guten Ausbeuten in die Sulfoniumsalze der allgemeinen Formel II überführbar sind und in stabiler Form erhalten werden konnten. Besonders gut verläuft die Reaktion, wenn, bezogen auf die Halogencarbonsäure, 1 Äquivalent einer Base zugesetzt wird. Als Halogencarbonsäuren werden bevorzugt Halogenessigsäuren und ganz besonders bevorzugt Brom- und Chloressigsäure eingesetzt. Im Falle der Chloressigsäure kann zur Reaktionsbeschleunigung 1 - 20 Mol-% eines Alkaliiodids, bevorzugt Kaliumiodid zugesetzt werden. Als Basen werden Alkalialkoxide und Alkalihydroxide bevorzugt.

Die Cyclisierung von II nach I ist eine intramolekulare nucleophile Substitution und verläuft prinzipiell analog zu der der entsprechenden Sulfoniumsalze (US 5,484,946). Prinzipiell sind alle dem Fachmann geläufigen Varianten für diese Reaktion heranzuziehen. Bedingt durch die Unbeständigkeit der Carboxymethylsulfoniumsalze ist bei dieser Reaktion allerdings mit einer Reihe von Nebenprodukten, wie z. B. der Eliminierung oder Substitution der Abgangsgruppe oder der Decarboxylierung zu den entsprechenden Dimethylsulfoniumsalzen zu rechnen. Darum war es sehr überraschend, daß für die Cyclisierung Bedingungen gefunden werden konnten, die den Einsatz von relativ starken Basen - tert. Amine, Alkali- oder Erdalkalialkoxide, -hydroxide oder -carbonaten - in Gegenwart eines polaren protischen Lösungsmittels erlauben und trotzdem eine weitestgehend selektiv verlaufende Reaktionsführung gestatten. Diese sehr preiswerten und gut für einen großtechnischen Prozess einsetzbaren unkritischen Reagenzien sind anderen Bedingungen aus dem Stand der Technik für die intramolekulare Substitution klar vorzuziehen (kein Natriumhydrideinsatz, keine teuren Reagenzien und Lösungsmittel). Die bei der Cyclisierung freigesetzte Methylmercaptocarbonsäuren haben einen sehr geringen Dampfdruck, inbesondere wenn sie in der Salzform vorliegen und führen daher, im Gegensatz zur Freisetzung von Dimethylsulfid aus den Dimethylsulfoniumsalzen, zu keiner nennenswerten Geruchsbelästigung.

Weiterhin vorteilhaft bei diesen bevorzugten Umsetzungsbedingungen ist, daß die optische Aktivität von enantiomer angereicherten Ausgangsverbindungen unter den erfindungsgemäßen Bedingungen nahezu vollständig erhalten bleibt. Wie der Vergleichsversuch zu der in der US 5,484,946 beschriebenen Variante zeigt, tritt teilweise sogar noch weniger Racemisierung ein (siehe Beispiele).

Bevorzugt kommen in der eben dargestellten Reaktion Derivate von II zum Einsatz, die als Rest R¹ die p-Cyanophenyl- oder p-Carbamoylphenylgruppe, für R² H und für R³ einen Benzyloxycarbonyl-Rest beinhalten. Diese führen zu vorteilhaften Verbindungen für die Herstellung von Pharmazeutika wie z. B. in der WO 94/22820 beschrieben.

Als Lösungsmittel für die Umwandlung II in I können alle organischen Lösungsmittel verwendet werden, die unter den gegebenen Bedingungen inert reagieren. Besonders bevorzugt sind Alkohole, wie z. B. Methanol, Ethanol, Ether, wie z. B. Methyl-tert.-butylether, Tetrahydrofuran, Dimethoxyethan, Dioxan, Kohlenwasserstoffe, wie z. B. Hexan, Cyclohexan, Toluol, Ketone, wie z. B. Aceton, Diethylketon, Methylisobutylketon, Nitrile, wie z. B. Acetonitril und Carbonsäurealkylester, wie z. B. Ethylacetat, Isopropylacetat, n-Butyllacetat. Ganz besonders bevorzugt werden die Lösungsmittel bzw. die Gemische eingesetzt, in denen die Sulfoniumsalze der allgemeinen Formel II hergestellt werden.

Als linear oder verzweigte (C₁-C₈)-Alkylreste sind anzusehen Methyl, Ethyl, n-Propyl, Isopropyl, n-Butyl, Isobutyl, sec-Butyl, tert.-Butyl, Pentyl, Hexyl, Heptyl oder Octyl samt aller ihrer Bindungsisomeren. Der linear oder verzweigte (C₂-C₈)-Alkenylrest umfaßt alle Substituenten, welche eben beim (C₁-C₈)-Alkylrest aufgezählt wurden mit Ausnahme vom Methylrest, wobei in diesen Resten mindestens eine Doppelbindung vorhanden ist. Der Umfang von (C₂-C₈)-Alkinyl entspricht dem von (C₂-C₈)-Alkenyl, wobei hier allerdings mindestens eine Dreifachbindung vorliegen muß. Der Rest (C₁-C₈)-Alkoxy entspricht dem Rest (C₁-C₈)-Alkyl mit der Maßgabe, daß dieser über ein Sauerstoffatom an den Cyclus gebunden ist. Als (C₂-C₈)-Alkoxyalkyl sind Reste gemeint, bei denen die Alkylkette durch mindestens eine Sauerstoffunktion unterbrochen ist, wobei nicht zwei Sauerstoffe miteinander verbunden sein können. Die Anzahl der Kohlenstoffatome gibt die Gesamtzahl der im Rest enthaltenen Kohlenstoffatome an. Als (C₂-C₈)-Alkenyloxy versteht man Reste wie (C₂-C₈)-Alkoxy, welche jedoch mindestens eine C-C-Doppelbindung aufweisen.
N-, O-, P-, S-atomhaltige Reste sind insbesondere Alkyl, Alkenyl, Alkinyl-Reste der oben genannten Art, welche eines oder mehrere dieser Heteroatome in ihrer Kette aufweisen bzw. welche über eines dieser Heteroatome an das Molekül gebunden sind. Unter (C₃-C₇)-Cycloalkyl versteht man Cyclopropyl, Cyclobutyl, Cyclopentyl, Cyclohexyl bzw. Cycloheptylreste.

Als Halogene kommen Fluor, Chlor, Brom und Iod in Frage.

Unter N-verknüpftem Aminosäure- oder Peptidrest wird eine Verbindung angesehen, bei der das in Frage stehende Molekül über sein N-Atom an das α-C-Atom einer sich von einer Aminosäure ableitenden Carbonsäure, welche Bestandteil eines Peptidrestes sein kann, gebunden ist.

Unter C-verknüpftem Aminosäure- oder Peptidrest wird eine Verbindung angesehen, bei der das in Frage stehende Molekül über sein N-Atom an das C-Atom der Carboxylgruppe einer Aminosäure, welche Bestandteil eines Peptids sein kann, gebunden ist.

Unter (C₁-C₈)-Acyl versteht man einen Alkylrest mit einem bis acht C-Atomen, der linear oder verzweigt vorliegen kann, also ebenfalls die gesamten möglichen Bindungsisomere mitumfaßt, welcher über eine C=O-Funktion an das Molekül gebunden ist.

Unter (C₇-C₁₅)-Arylalkyl versteht man einen Alkylrest, der mit einem Arylrest, wie beispielsweise Phenyl, verbunden ist. Phenyl kann dabei den Anfang oder das Ende des Alkylrests bilden oder ihn unterbrechen.

Verbindungen mit einem stereogenen Zentrum bedeuten im Rahmen der Erfindung Racemate sowie die jeweiligen enantiomeren Antipoden dieser Strukturen. Für das erfindungsgemäße Verfahren bevorzugt ist allerdings der Einsatz enantiomerenangereicherter Methioninderivate der allgemeinen Formel III. So erhält man bei Einsatz der enantiomerenangereicherten Edukte (D **oder** L) unter nahezu vollständiger Stereokonservation die enantiomerenangereicherten Produkte der allgemeinen Formel V und I (D **oder** L).

Unter Säureadditionssalzen versteht man ionische Additionsverbindungen aus starken Säuren wie HCl, HBr, H₂SO₄, H₃PO₄, CF₃COOH, p-Toluolsulfonsäure, Methansulfonsäure und dem betrachteten Molekül.

Die folgenden Beispiele sollen die vorliegende Erfindung erläutern.

### Beispiele:

**1) Darstellung des N-Benzyloxocarbonyl-S-carboxymethyl-Lmethionin-4-cyanophenylanilids**
   **a) mit Bromessigsäure**
      402.4 g (1.04 mol) N-Benzyloxocarbonyl-L-methionin-4-cyanophenylanilid werden in 600 ml Methanol bei Raumtemperatur suspendiert und anschließend unter Refluxion erwärmt. Daraufhin werden parallel 217.4 g (1.56 mol) Bromessigsäure, gelöst in 600 ml Wasser, sowie 86 ml (1.64 mol) 50%-ige Natronlauge zugetropft. Die nun klare Reaktionsmischung wird für 1 h unter Refluxion gerührt und anschließend abgekühlt. Nach einigen Tagen fällt ein weißer Niederschlag aus, der abgetrennt und dreimal mit 100 ml Wasser gewaschen wird. Nach Trocknung erhält man 269 g (47 %) eines weißen Feststoffes.
      1H-NMR (DMSO): 2.1 (m, 1H), 2.4 (m, 1H), 3.1 (m, 3H), 3.5 (m, 2H), 4.3 (m, 2H), 4.4 (m, 2H), 5.1 (m, 2H), 7.3 (m, 5H), 7,8 (m, 2H), 7.9 (m, 2H), 11.3 (m, 1H).
      Schmp.: 124 - 129 °C (Zers.)
   b) mit Chloressigsäure / Kaliumiodid
      14.0 g (35.8 mmol) N-Benzyloxocarbonyl-L-methionin-4-cyanophenylanilid und 0.15 g (0.9 mmol) Kaliumiodid werden in 14 ml Aceton bei 70°C suspendiert und innerhalb von 30 min mit einer Lösung von 4.1 g (43 mmol) Chloressigsäure versetzt. Nach weiteren 30 min sind im HPLC lediglich 4,6 Fl.-% des 0 N-Benzyloxocarbonyl-S-carboxymethyl-L-methionin-4-cyanophenylanilids neben 94.8 Fl-% des Eduktes nachweisbar. Nach Einstellen des pH-Wertes auf 5.0 durch Zugabe von 50%-iger Natronlauge sind nach 1 Stunde bereits 21 Fl.-% und nach 2 Stunden 35 Fl.-% Produkt entstanden.
**2) Darstellung des (S)-3-Benzyloxocarbonylamino-1-(4-cyanophenyl)-2-pyrrolidinon in Acetonitril mit wäßriger Natronlauge**
   25.0 g (46 mmol) N-Benzyloxocarbonyl-S-carboxymethyl-Lmethionin-4-cyanophenylanilid werden in 45 ml Acetonitril bei Raumtemperatur suspendiert. Innerhalb von 5 min werden 2.3 ml (44 mmol) 50%-ige Natronlauge zugetropft und die Reaktionsmischung für 3 h gerührt. Anschließend werden erneut 0.69 ml (13.2 mmol) 50%-ige Natronlauge zugefügt und der Ansatz für weitere 3 h gerührt. Zur Aufarbeitung werden 45 ml Wasser zugesetzt und der pH-Wert auf 6 gestellt. Der Niederschlag wird abgetrennt. Man erhält nach Trocknung 13.1 g (90%) eines weißen Feststoffes.
   Schmp: 192 - 196 °C
   1H-NMR (DMSO): 2.02 (m, 1H), 2.41 (m, 1H) , 3.78 (m, 1H), 3.84 (m, 1H), 4.47 (m, 1H), 5.07 (s, 2H), 7.33, 7.38 (m, 5H), 7.77 (d, 1H), 7.88 (dd, 4H).
   D-Anteil(HPLC) : 7.2 %
**3) Darstellung des (S)-3-Benzyloxocarbonylamino-1-(4-cyanophenyl)-2-pyrrolidinons ohne Isolierung des N-Benzyloxocarbonyl-S-carboxymethyl-L-methionin-4-cyanophenylanilids**
   **a) mit Bromessigsäure in Methanol und wäßriger Natronlauge / Kaliumcarbonat**
      38.6 g (100 mmol) N-Benzyloxocarbonyl-L-methionin-4-cyanophenylanilid werden in 60 ml Methanol suspendiert und auf 60°C erwärmt. Anschließend werden 20.8 g (150 mmol) Bromessigsäure, gelöst in 50 ml Methanol, sowie parallel 6 ml (114 mmol) 50%-Natronlauge innerhalb von 1.75 h zugetropft. Die klare Reaktionsmischung wird für weitere 2 h bei 60°C gerührt. Nach Abkühlung auf Raumtemperatur (RT) wird der Ansatz geteilt, mit 6.9 g (50 mmol) Kaliumcarbonat versetzt und für 4 h auf 60 °C erwärmt. Nach Abkühlung auf Raumtemperatur (RT) werden 75 ml Wasser zugesetzt und nach Zugabe von konz. Salzsäure ein pH-Wert von 1.5 eingestellt. Der Niederschlag wird abfiltriert und zweimal mit 10 ml Wasser gewaschen. Man erhält nach Trocknung 12.5 g (75 %) eines weißen Feststoffes
      D-Anteil (HPLC): 11.5 %
   **b) mit Bromessigsäure in Methanol und Methylisobutylketon/Acetonitril und wäßriger Natronlauge**
      11.0 g (28.5 mmol) N-Benzyloxocarbonyl-L-methionin-4-cyanophenylanilid werden in 15 ml Methanol suspendiert und auf 60°C erwärmt. Anschließend werden 6.06 g (42.75 mmol) Bromessigsäure, gelöst in 15 ml Methanol, sowie parallel 2.3 ml (42.75 mmol) 50%-ige Natronlauge innerhalb von 70 min. zugetropft. Die klare Reaktionsmischung wird für weitere 2 h bei 60°C gerührt. Nach Abkühlung auf RT und Neutralisation werden 100 ml Methylisobutylketon zugesetzt und anschließend bei leichtem Vakuum 35 ml abdestilliert. Bei Abkühlung auf Raumtemperatur wurden 15 ml Acetonitril zugefügt und die Suspension daraufhin mit 1.3 ml (28 mmol) 50%-iger Natronlauge versetzt und für 1.5 h bei RT gerührt. Der Niederschlag wurde abfiltriert und zweimal mit 10 ml Wasser gewaschen. Man erhält nach Trocknung 4.5 g (46 %) eines weißen Feststoffes.
      D-Anteil (HPLC): 4.4 %
**c) mit Bromessigsäure in Methanol und Kaliumhydroxid und Kaliumcarbonat**
   Zu einer Suspension von 19.3 g (50 mmol) N-Benzyloxocarbonyl-L-methionin-4-cyanophenylanilid und 10.4 g (75 mmol) Bromessigsäure in 35 ml Methanol wird bei 60°C eine Lösung von 5.0 g (75 mmol) 85%-igem Kaliumhydroxid in 15 ml Methanol gegeben. Nach 4 h bei 60°C werden 6.9 g (50 mmol) Kaliumcarbonat zugegeben und für 3 h auf 60°C erwärmt. Nach Zugabe von 65 ml Wasser und Ansäuern mit konz. Salzsäure auf pH 1 wird der Niederschlag abgesaugt, mit Wasser gewaschen und getrocknet. Man erhält 14.5 g (86%) eines weißen Feststoffes.
   D-Anteil (HPLC) : 12.3 %
**d) mit Bromessigsäure in Methanol und Kaliumhydroxid**
   Der vorhergehende Ansatz wird mit der Maßgabe wiederholt, daß anstelle des Kaliumcarbonats bezogen auf N-Benzyloxocarbonyl-L-methionin-4-cyanophenylanilid 1.5 Äquivalente 85%-iges Kaliumhydroxid eingesetzt werden und die Reaktionszeit nur 30 min beträgt. Man erhält 14.0 g (84%) eines weißen Feststoffes.
   D-Anteil (HPLC): 8.4 %
**e) mit Bromessigsäure in Methanol und Kaliumhydroxid sowie Natriummethylat**
   5.0 g (75 mmol) Kaliumhydroxid werden in 50 ml Methanol gelöst und mit 10.4 g (75 mmol) Bromessigsäure versetzt. Nach Zugabe von 19.3 g (50 mmol) N-Benzyloxocarbonyl-L-methionin-4-cyanophenylanilid wird 3 h auf 60°C erhitzt und dann 63 ml (55 mmol) einer 0.87 M Natriummethylatlösung in Methanol zugegeben. Nach 40 min stellt man den pH-Wert mit konz. Salzsäure auf 1.5 und kühlt auf 25°C ab. Der Niederschlag wird abfiltriert, mit Wasser gewaschen und getrocknet. Man erhält 9.3 g (56%) eines weißen Feststoffs.
   D-Anteil (HPLC): 9.8 %
**f) mit Chloressigsäure/ Kaliumiodid in Methanol/Methylisobutylketon und wäßriger Natronlauge**
   Zu 10.0 g (26.1 mmol) N-Benzyloxocarbonyl-L-methionin-4-cyanophenylanilid in 15 ml Methanol wird bei Rückflußtemperatur innerhalb von 30 min eine Lösung von 2.71 g (28.7 mmol)Chloressigsäure und 1.1 g Kaliumiodid in 15 ml Wasser zugetropft. Nach 2 h werden weitere 1.3 g Chloressigsäure zugegeben und der pH-Wert mit 50%-iger Natronlauge auf 5.5 gestellt. Nach 8 h wird der pH-Wert auf 7.1 gestellt und mit 100 ml Methylisobutylketon versetzt. Danach werden bei 70°C i.Vak. 13 ml Wasser azeotrop abdestilliert und der Rückstand bei Raumtemperatur mit 1.2 ml (26 mmol) 50-%iger Natronlauge versetzt. Nach 2 h wird der pH-Wert auf 7 gestellt und der Niederschlag abfiltriert. Man erhält 5.34 g eines 70%-igen Produktes.
   D-Anteil (HPLC): 3.3 %
**4) Herstellung des 3-Amino-1-(4-cyanophenyl)-2-oxopyrrolidinhydrochlorids**
   In eine Suspension von 50 g (0.15 mol) 3-Benzyloxocarbonyl-amino-1-(4-cyanophenyl)-2-oxopyrrolidin in 600 ml Methanol werden 6.0 g (0.16 mol) gasförmige Salzsäure eingeleitet. Nach Zugabe von 2.5 g 5 % Palladium auf A-Kohle wird bei 45 °C Wasserstoff durchgeleitet. Nach 40 min wird abgekühlt, der Katalysator abfiltriert und das Filtrat zur Trockene eingeengt. Nach Umkristallisation aus Methanol/Isopropanol erhält man 28.5 g (80 %) 3-Amino-1-(4-cyanophenyl)-2-oxo-pyrrolidin Hydrochlorid.
   Schmp: 256 - 258 °C (Zers.)
   1H-NMR (DMSO): 2.20 (m, 1H), 2.55 (m, 1H), 3.87 (dt, 1H), 3.97 (t, 1H); 4.27 (dd, 1H), 7.91 (s, 4H), 8.89 (s, 3H).
**5) Vergleichsversuch: Cyclisierung des N-Benzyloxocarbonyl-S-methyl-L-methionin-4-cyanophenylanilids solfoniumsalzes in DMSO**
   3.12 g (8.2 mmol) N-Benzyloxocarbonyl-L-methionin-4-cyanophenylanilid werden in 6 ml DMSO gelöst , mit 5.0 g (24.5 mmol) Trimethylsulfoniumiodid und 1.69 g (12.2 mmol) Kaliumcarbonat versetzt und 3 h auf 80°C erhitzt. Danach gibt man 30 ml Wasser zu, filtriert den Niederschlag ab und wäscht mit Wasser nach. Nach Trocknen erhält man 2.0 g (72%).
   D-Anteil (HPLC): 9.2 %

## Patentansprüche

1. Verfahren zur Herstellung von γ-Lactamen der allgemeinen Formel I und deren Salze 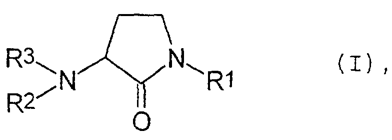 worin:
R¹ bedeutet H, (C₁-C₈)-Alkyl, (C₂-C₈)-Alkenyl, (C₂-C₈)-Alkinyl, (C₂-C₈)-Alkoxyalkyl, (C₁-C₈)-Acyl, welche gegebenenfalls linear oder verzweigt vorliegen können sowie einfach oder mehrfach mit Halogenen, N-, O-, P-, S-atomhaltigen Resten substituiert sein können, (C₃-C₇)-Cycloalkyl, das gesättigt oder ungesättigt sowie einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein kann oder im Ring Heteroatome wie N, O, P, S enthalten kann, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die genannten Cyclen gegebenenfalls einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, mit Nitril, mit C(O)NH₂ oder N-, O-, P-, S-atomhaltigen Resten substituiert sein können, N-verknüpfter Aminosäure- oder Peptidrest,
R² bedeutet H, (C₁-C₈)-Alkyl, (C₂-C₈)-Alkenyl, (C₂-C₈)-Alkinyl, (C₂-C₈)-Alkoxyalkyl, welche gegebenenfalls linear oder verzweigt vorliegen können sowie einfach oder mehrfach mit Halogenen, N-, O-, P-, S-atomhaltigen Resten substituiert sein können, (C₃-C₇)-Cycloalkyl, das gesättigt oder ungesättigt sowie einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein kann und/oder im Ring Heteroatome wie N, O, P, S enthalten können, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die genannten Cyclen gegebenenfalls einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen oder N-, O-, P-, S-atomhaltigen Resten substituiert sein können,
R³ bedeutet H, (C₁-C₈)-Acyl, das gegebenenfalls linear oder verzweigt vorliegen kann,
ein C-verknüpfter Aminosäure- oder Peptidrest oder eine gängige Peptidschutzgruppe wie z. B. Formyl, Carbamoyl, Benzyloxycarbonyl, tert.-Butyloxycarbonyl, Allyloxycarbonyl, Trifluoracetyl,
**dadurch gekennzeichnet,**
**daß** man Derivate der allgemeinen Formel II 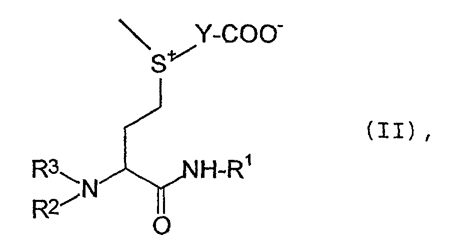 worin R¹, R², R³ die oben angegebene Bedeutung besitzen und Y für einen linearen oder verzweigten (C₁-C₈)-Alkylenrest, der gegebenenfalls mehrfach mit (C₁-C₄)-Alkyl, Halogen, Hydroxy oder Phenyl substituiert sein kann, (C₇-C₁₅)-Arylalkyl, das gegebenenfalls mehrfach mit (C₁-C₄)-Alkyl, Halogen oder Hydroxy substituiert sein kann, zu Verbindungen der allgemeinen Formel I cyclisiert.

2. Verfahren nach Anspruch 1,
**dadurch gekennzeichnet,**
**daß** man unter basischen Bedingungen, vorzugsweise mit Kaliumhydroxid oder Kaliumcarbonat, cyclisiert.

3. Verfahren nach den Ansprüchen 1 oder 2,
**dadurch gekennzeichnet,**
**daß** man die Cyclisierung in einem polaren protischen Lösungsmittel durchführt.

4. Verfahren nach Anspruch 3,
**dadurch gekennzeichnet,**
**daß** man die Cyclisierung in Methanol durchführt.

5. Verfahren nach einem oder mehreren der Ansprüche 1-4,
**dadurch gekennzeichnet,**
**daß** Y für -CH₂- steht.

6. Verfahren nach einem oder mehreren der Ansprüche 1-5,
**dadurch gekennzeichnet,**
**daß** man als Verbindung II eine Verbindung einsetzt, bei der R¹ gleich p-Cyanophenyl oder p-Carbamoylphenyl,
R² gleich H und R³ gleich Benzyloxycarbonyl ist.

7. Verfahren nach einem oder mehreren der Ansprüche 1-6,
**dadurch gekennzeichnet,**
**daß** man die Cyclisierung bei Temperaturen von 0 bis 150 °C betreibt.

8. Verfahren nach einem oder mehreren der Ansprüche 1-7,
**dadurch gekennzeichnet,**
**daß** man Verbindungen der allgemeinen Formel II aus Derivaten der allgemeinen Formel III 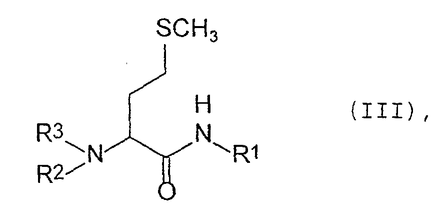 worin R¹, R², R³ die oben angegebene Bedeutung besitzen, durch Umsetzung mit Halogencarbonsäuren der allgemeinen Formel IV
X-Y-COOH (IV)
worin Y die oben angebenene Bedeutung besitzt und X für ein Halogen steht, herstellt.

9. Verfahren nach Anspruch 8,
**dadurch gekennzeichnet,**
**daß** die Umsetzung in Gegenwart einer Base durchgeführt wird.

10. Verfahren nach Anspruch 9,
**dadurch gekennzeichnet,**
**daß** als Base ein Alkalihydroxid, vorzugsweise Kaliumhydroxid, eingesetzt wird.

11. Verfahren nach einem oder mehreren der Ansprüche 8-10,
**dadurch gekennzeichnet,**
**dass** als Halogencarbonsäure der allgemeinen Formel IV eine α-Halogencarbonsäure, vorzugsweise eine α-Halogenessigsäure, eingesetzt wird.

12. Verfahren nach Anspruch 11,
**dadurch gekennzeichnet,**
**daß** Bromessigsäure oder Chloressigsäure als α-Halogencarbonsäure verwendet wird.

13. Verfahren nach einem oder mehreren der Ansprüche 8-12,
**dadurch gekennzeichnet,**
**daß** die Umsetzung bei Temperaturen von -20°C bis 150°C durchgeführt wird.

14. Verfahren nach einem oder mehreren der Ansprüche 8-13,
**dadurch gekennzeichnet,**
**daß** die Umsetzung in polaren protischen Lösungsmitteln, vorzugsweise Methanol, durchgeführt wird.

15. Verfahren nach einem oder mehreren der Ansprüche 1-14,
**dadurch gekennzeichnet,**
**daß** man die Verbindung der allgemeinen Formel II ohne vorherige Isolierung zur Verbindung der allgemeinen Formel I cyclisiert.

16. Verbindungen der allgemeinen Formel II und deren Säureadditionssalze 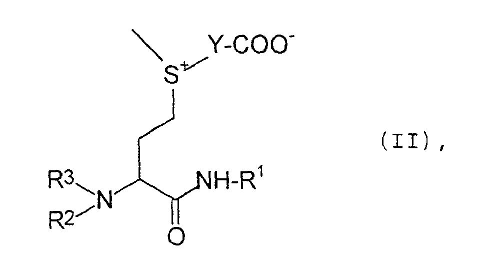 worin:
R¹ bedeutet H, (C₁-C₈)-Alkyl, (C₂-C₈)-Alkenyl, (C₂-C₈)-Alkinyl, (C₂-C₈)-Alkoxyalkyl, (C₁-C₈)-Acyl, welche gegebenenfalls linear oder verzweigt vorliegen können sowie einfach oder mehrfach mit Halogenen, N-, O-, P-, S-atomhaltigen Resten substituiert sein können, (C₃-C₇)-Cycloalkyl, das gesättigt oder ungesättigt sowie einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein kann oder im Ring Heteroatome wie N, O, P, S enthalten kann, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die genannten Cyclen gegebenenfalls einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, mit Nitril, mit C(O)NH₂ oder N-, O-, P-, S-atomhaltigen Resten substituiert sein können,
R² bedeutet H, (C₁-C₈)-Alkyl, (C₂-C₈)-Alkenyl, (C₂-C₈)-Alkinyl, (C₂-C₈)-Alkoxyalkyl, welche gegebenenfalls linear oder verzweigt vorliegen können sowie einfach oder mehrfach mit Halogenen, N-, O-, P-, S-atomhaltigen Resten substituiert sein können, (C₃-C₇)-Cycloalkyl, das gesättigt oder ungesättigt sowie einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein kann und/oder im Ring Heteroatome wie N, O, P, S enthalten können, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die genannten Cyclen gegebenenfalls einfach oder mehrfach mit linearem oder verzweigtem (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen oder N-, O-, P-, S-atomhaltigen Resten substituiert sein können,
R³ bedeutet H, (C₁-C₈)-Acyl, das gegebenenfalls linear oder verzweigt vorliegen kann, eine gängige Peptidschutzgruppe wie z. B. Formyl, Carbamoyl, Benzyloxycarbonyl, tert.-Butyloxycarbonyl, Allyloxycarbonyl, Trifluoracetyl, und
Y für einen linearen oder verzweigten (C₁-C₈)-Alkylenrest, der gegebenenfalls mehrfach mit (C₁-C₄)-Alkyl, Halogen, Hydroxy oder Phenyl substituiert sein kann, (C₇-C₁₅)-Arylalkyl, das gegebenenfalls mehrfach mit (C₁-C₄)-Alkyl, Halogen oder Hydroxy substituiert sein kann, steht.

17. Verbindungen der allgemeinen Formel II gemäß Anspruch 16 mit R² gleich H, R³ gleich Benzyloxycarbonyl und Y gleich -CH₂-.

18. Verbindungen der allgemeinen Formel II gemäß Anspruch 16 mit R² gleich H, R³ gleich Benzyloxycarbonyl, Y gleich -CH₂- und R¹ gleich p-Cyanophenyl oder p-Carbamoylphenyl.

19. Verwendung der Verbindungen gemäß der Ansprüche 16-18 zur Herstellung von Intermediaten für bioaktive Wirkstoffe.

## Claims

1. Process for the preparation of γ-lactams having the general formula I and salts thereof 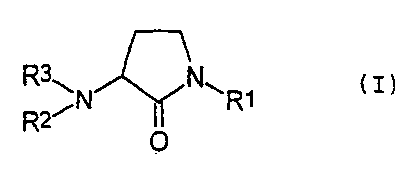 wherein:
R¹ denotes H, (C₁-C₈) alkyl, (C₂-C₈) alkenyl, (C₂-C₈) alkynyl, (C₂-C₈) alkoxy alkyl, (C₁-C₈) acyl, which can optionally be linear or branched and can be monosubstituted or polysubstituted with halogens, N, O, P, S atom-containing radicals, (C₃-C₇) cycloalkyl, which can be saturated or unsaturated and can be monosubstituted or polysubstituted with linear or branched (C₁-C₈) alkyl, (C₂-C₈) alkenyl, (C₂-C₈) alkynyl, (C₁-C₈) acyl, (C₁-C₈) alkoxy, (C₂-C₈) alkoxy alkyl, with halogens, N, O, P, S atom-containing radicals or can contain heteroatoms such as N, O, P, S in the ring, aryl, such as phenyl or naphthyl, aralkyl, such as benzyl or phenethyl, heteroaryl, such as furyl, pyrrolyl, pyridyl, heteroaralkyl, such as furfuryl, pyrrolyl methyl, pyridyl methyl, furyl ethyl, pyrrolyl ethyl, pyridyl ethyl, whereby the cited cyclic compounds can optionally be monosubstituted or polysubstituted with linear or branched (C₁-C₈) alkyl, (C₂-C₈) alkenyl, (C₂-C₈) alkynyl, (C₁-C₈) acyl, (C₁-C₈) alkoxy, (C₂-C₈) alkoxy alkyl, with halogens, with nitrile, with C(O)NH₂ or N, O, P, S atom-containing radicals, N-bonded amino acid or peptide radical,
R² denotes H, (C₁-C₈) alkyl, (C₂-C₈) alkenyl, (C₂-C₈) alkynyl, (C₂-C₈) alkoxy alkyl, which can optionally be linear or branched and can be monosubstituted or polysubstituted with halogens, N, O, P, S atom-containing radicals, (C₃-C₇) cycloalkyl, which can be saturated or unsaturated and can be monosubstituted or polysubstituted with linear or branched (C₁-C₈) alkyl, (C₂-C₈) alkenyl, (C₂-C₈) alkynyl, (C₁-C₈) acyl, (C₁-C₈) alkoxy, (C₂-C₈) alkoxy alkyl, with halogens, N, O, P, S atom-containing radicals and/or can contain heteroatoms such as N, O, P, S in the ring, aryl, such as phenyl or naphthyl, aralkyl, such as benzyl or phenethyl, heteroaryl, such as furyl, pyrrolyl, pyridyl, heteroaralkyl, such as furfuryl, pyrrolyl methyl, pyridyl methyl, furyl ethyl, pyrrolyl ethyl, pyridyl ethyl, whereby the cited cyclic compounds can optionally be monosubstituted or polysubstituted with linear or branched (C₁-C₈) alkyl, (C₂-C₈) alkenyl, (C₂-C₈) alkynyl, (C₁-C₈) acyl, (C₁-C₈) alkoxy, (C₂-C₈) alkoxy alkyl, with halogens or N, O, P, S atom-containing radicals,
R³ denotes H, (C₁-C₈) acyl, which can optionally be linear or branched, a C-bonded amino acid or peptide radical or a conventional peptide protective group such as e.g. formyl, carbamoyl, benzyl oxycarbonyl, tert.-butyl oxycarbonyl, allyl oxycarbonyl, trifluoroacetyl,
**characterised in that**
derivatives having the general formula II 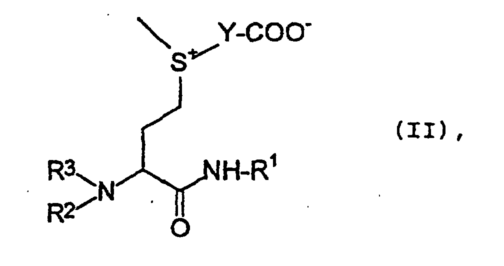 wherein R¹, R², R³ have the meaning cited above and Y stands for a linear or branched (C₁-C₈) alkylene radical, which can optionally be polysubstituted with (C₁-C₄) alkyl, halogen, hydroxy or phenyl, (C₇-C₁₅) aryl alkyl, which can optionally be polysubstituted with (C₁-C₄) alkyl, halogen or hydroxy, are cyclised to compounds having the general formula I.

2. Process according to claim 1,
**characterised in that**
cyclisation is performed under basic conditions, preferably with potassium hydroxide or potassium carbonate.

3. Process according to claims 1 or 2,
**characterised in that**
the cyclisation is performed in a polar protic solvent.

4. Process according to claim 3,
**characterised in that**
the cyclisation is performed in methanol.

5. Process according to one or more of claims 1 to 4,
**characterised in that**
Y stands for -CH₂-.

6. Process according to one or more of claims 1 to 5,
**characterised in that**
a compound in which R¹ is equal to p-cyanophenyl or p-carbamoyl phenyl, R² is equal to H and R³ is equal to benzyl oxycarbonyl, is used as compound II.

7. Process according to one or more of claims 1 to 6,
**characterised in that**
the cyclisation is run at temperatures of 0 to 150 °C.

8. Process according to one or more of claims 1 to 7,
**characterised in that**
compounds having the general formula II are prepared from derivatives having the general formula III 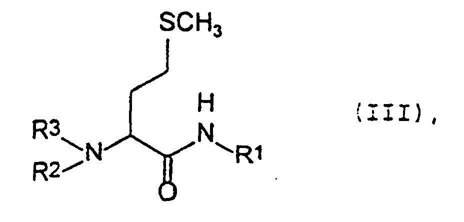 wherein R¹, R², R³ have the meaning cited above, by reaction with haloacids having the general formula IV
X-Y-COOH (IV),
wherein Y has the meaning cited above and X stands for a halogen.

9. Process according to claim 8,
**characterised in that**
the reaction is performed in the presence of a base.

10. Process according to claim 9,
**characterised in that**
an alkali hydroxide, preferably potassium hydroxide, is used as the base.

11. Process according to one or more of claims 8 to 10,
**characterised in that**
an α-haloacid, preferably an α-haloacetic acid, is used as the haloacid having the general formula IV.

12. Process according to claim 11,
**characterised in that**
bromoacetic acid or chloroacetic acid is used as the α-haloacid.

13. Process according to one or more of claims 8 to 12,
**characterised in that**
the reaction is performed at temperatures of -20°C to 150°C.

14. Process according to one or more of claims 8 to 13,
**characterised in that**
the reaction is performed in polar protic solvents, preferably methanol.

15. Process according to one or more of claims 1 to 14,
**characterised in that**
the compound having the general formula II is cyclised to the compound having the general formula I without prior isolation.

16. Compounds having the general formula II and acid addition salts thereof 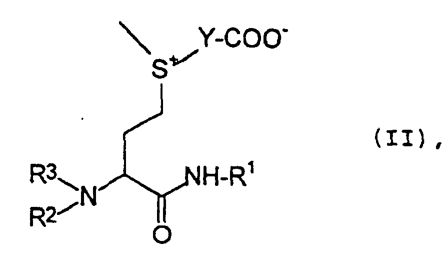 wherein:
R¹ denotes H, (C₁-C₈) alkyl, (C₂-C₈) alkenyl, (C₂-C₈) alkynyl, (C₂-C₈) alkoxy alkyl, (C₁-C₈) acyl, which can optionally be linear or branched and can be monosubstituted or polysubstituted with halogens, N, O, P, S atom-containing radicals, (C₃-C₇) cycloalkyl, which can be saturated or unsaturated and can be monosubstituted or polysubstituted with linear or branched (C₁-C₈) alkyl, (C₂-C₈) alkenyl, (C₂-C₈) alkynyl, (C₁-C₈) acyl, (C₁-C₈) alkoxy, (C₂-C₈) alkoxy alkyl, with halogens, N, O, P, S atom-containing radicals or can contain heteroatoms such as N, O, P, S in the ring, aryl, such as phenyl or naphthyl, aralkyl, such as benzyl or phenethyl, heteroaryl, such as furyl, pyrrolyl, pyridyl, heteroaralkyl, such as furfuryl, pyrrolyl methyl, pyridyl methyl, furyl ethyl, pyrrolyl ethyl, pyridyl ethyl, whereby the cited cyclic compounds can optionally be monosubstituted or polysubstituted with linear or branched (C₁-C₈) alkyl, (C₂-C₈) alkenyl, (C₂-C₈) alkynyl, (C₁-C₈) acyl, (C₁-C₈) alkoxy, (C₂-C₈) alkoxy alkyl, with halogens, with nitrile, with C(O)NH₂ or N, O, P, S atom-containing radicals,
R² denotes H, (C₁-C₈) alkyl, (C₂-C₈) alkenyl, (C₂-C₈) alkynyl, (C₂-C₈) alkoxy alkyl, which can optionally be linear or branched and can be monosubstituted or polysubstituted with halogens, N, O, P, S atom-containing radicals, (C₃-C₇) cycloalkyl, which can be saturated or unsaturated and can be monosubstituted or polysubstituted with linear or branched (C₁-C₈) alkyl, (C₂-C₈) alkenyl, (C₂-C₈) alkynyl, (C₁-C₈) acyl, (C₁-C₈) alkoxy, (C₂-C₈) alkoxy alkyl, with halogens, N, O, P, S atom-containing radicals and/or can contain heteroatoms such as N, O, P, S in the ring, aryl, such as phenyl or naphthyl, aralkyl, such as benzyl or phenethyl, heteroaryl, such as furyl, pyrrolyl, pyridyl, heteroaralkyl, such as furfuryl, pyrrolyl methyl, pyridyl methyl, furyl ethyl, pyrrolyl ethyl, pyridyl ethyl, whereby the cited cyclic compounds can optionally be monosubstituted or polysubstituted with linear or branched (C₁-C₈) alkyl, (C₂-C₈) alkenyl, (C₂-C₈) alkynyl, (C₁-C₈) acyl, (C₁-C₈) alkoxy, (C₂-C₈) alkoxy alkyl, with halogens or N, O, P, S atom-containing radicals,
R³ denotes H, (C₁-C₈) acyl, which can optionally be linear or branched, a conventional peptide protective group such as e.g. formyl, carbamoyl, benzyl oxycarbonyl, tert.-butyl oxycarbonyl, allyl oxycarbonyl, trifluoroacetyl, and
Y stands for a linear or branched (C₁-C₈) alkylene radical, which can optionally be polysubstituted with (C₁-C₄) alkyl, halogen, hydroxy or phenyl, (C₇-C₁₅) aryl alkyl, which can optionally be polysubstituted with (C₁-C₄) alkyl, halogen or hydroxy.

17. Compounds having the general formula II according to claim 16, where R² equals H, R³ equals benzyl oxycarbonyl and Y equals -CH₂-.

18. Compounds having the general formula II according to claim 16, where R² equals H, R³ equals benzyl oxycarbonyl, Y equals -CH₂- and R¹ equals p-cyanophenyl or p-carbamoyl phenyl.

19. Use of the compounds according to claims 16 to 18 to prepare intermediates for biologically active agents.

## Revendications

1. Procédé de préparation de composés de gamma-lactames de formule générale I et de leurs sels 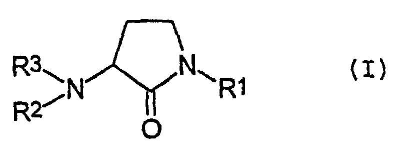 dans laquelle:
R¹ représente un alkyle en C₁ à C₈, un alcényle en C₂ à C₈, un alcynyle en C₂ à C₈, alcoxyalkyle en C₂ à C₈, un acyle en C₁ à C₈, qui le cas échéant peuvent se présenter sous forme linéaire ou ramifiée ainsi qu'être mono- ou polysubstitués avec les halogènes, des radicaux contenant des atomes de N, O, P, S, un cycloalkyle en C₃ à C₇; qui peut être saturé ou insaturé ainsi que mono- ou polysubstitué par des radicaux alkyle en C₁ à C₈, alcényle en C₂ à C₈, alcynyle en C₂ à C₈, acyle en C₁ à C₈, alcoxy en C₁ à C₈, alcoxyalkyle en C₂ à C₈ linéaire ou ramifié mono- ou polysubstitué par des radicaux contenant des halogènes, des atomes de N, O, P, S ou contenir dans le noyau des hétéroatomes tels que N, O, P, S, un aryle comme phényle ou naphtyle, un aralkyle comme benzyle ou phénéthyle, un hétéroaryle, comme furyle, pyrrolyle, pyridyle, un hétéroalkyle comme furfuryle, pyrrolylméthyle, piridineméthyle, furyléthyle, pyrrolyl éthyle, pyridyl éthyle, où les cycles mentionnés peuvent le cas échéant être mono- ou polysubstitués par des groupes alkyle en C₁ à C₈, alcényle en C₂ à C₈, alcynyle en C₂ à C₈, acyle en Ci à C₈, alcoxy en Ci à C₈, alcoxyalkyle en C₂ à C₈ linéaire ou ramifié, avec des halogènes, avec un nitrile, avec C(O)NH₂ ou des radicaux contenant N, O, P, S, un radical acide aminé ou peptide à ramification azotée,
R² représente un alkyle en C₁ à C₈, un alcényle en C₂ à C₈, un alcynyle en C₂ à C₈, alcoxyalkyle en C₂ à C₈, qui peuvent se présenter éventuellement sous forme linéaire ou ramifiée ainsi qu'être mono- ou polysubstitués avec les halogènes, des radicaux contenant des atomes de N, O, P, S, un cycloalkyle en C₃ à C₇; qui peut être saturé ou insaturé ainsi que mono- ou polysubstitué par des radicaux alkyle en C₁ à C₈, alcényle en C₂ à C₈, alcynyle en C₂ à C₈, acyle en C₁ à C₈, alcoxy en C₁ à C₈, alcoxyalkyle en C₂ à C₈ linéaire ou ramifié mono- ou polysubstitué par des radicaux contenant des halogènes, des atomes de N, O, P, S et/ou contenir dans le noyau des hétéroatomes tels que N, O, P, S, un aryle comme phényle ou naphtyle, un aralkyle comme benzyle ou phénéthyle, un hétéroaryle, comme furyle, pyrrolyle, pyridyle, un hétéroalkyle comme furfuryle, pyrrolylméthyle, piridineméthyle, furyléthyle, pyrrolyl éthyle, pyridyl éthyle, où les cycles mentionnés peuvent le cas échéant être mono- ou polysubstitués par des groupes alkyle en C₁ à C₈, alcényle en C₂ à C₈, alcynyle en C₂ à C₈, acyle en C₁ à C₈, alcoxy en C₁ à C₈, alcoxyalkyle en C₂ à C₈ linéaire ou ramifié, avec des halogènes ou des radicaux contenant N, O, P, S,
R³ représente H, un acyle en C₁ à C₈, qui peut être le cas échéant linéaire ou ramifié,
un radical acide aminé ou peptide à liaison carbonée ou un groupe protecteur de peptides courants, comme par exemple formyle, carbamoyle, benzyloxy carbonyle, tert.-butyloxycarbonyle, allyloxy carbonyle, trifluoroacétyle,
**caractérisé en ce que**
l'on cyclise des dérivés de formule générale II 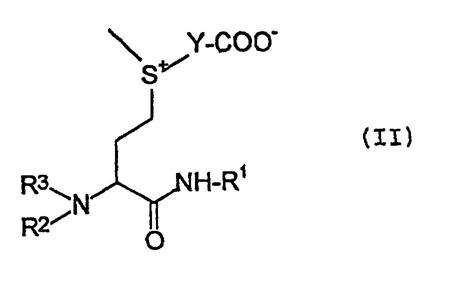 dans laquelle R¹, R², R³ possèdent la signification indiquée ci-dessus et Y représente un radical alkylène en C₁ à C₈ linéaire ou ramifié, qui le cas échéant peut être polysubstitué par un alkyle en C₁ à C₄, un hydroxy ou un phényle, un arylalkyle en C₇ à C₁₅, qui le cas échéant peut être polysubstitué par un alkyle en C₁ à C₄, un halogène ou un hydroxy, pour donner des composés de formule générale I.

2. Procédé selon la revendication 1,
**caractérisé en ce qu'**
on cyclise dans des conditions basiques, de préférence avec de l'hydroxyde de potassium ou du carbonate de potassium.

3. Procédé selon les revendications 1 ou 2,
**caractérisé en ce qu'**
on cyclisation dans un solvant protique polaire.

4. Procédé selon la revendication 3,
**caractérisé en ce qu'**
on conduit la cyclisation dans le méthanol.

5. Procédé selon une ou plusieurs des revendications 1 à 4,
**caractérisé en ce que**
Y représente -CH₂-.

6. Procédé selon une ou plusieurs des revendications 1 à 5,
**caractérisé en ce qu'**
on utilise comme composé II un composé dans lequel R¹ représente un p-cyanophényle ou un p-carbamoylphényle, R² représente H, R³ représente un benzyloxy carbonyle.

7. Procédé selon une ou plusieurs des revendications 1 à 6,
**caractérisé en ce qu'**
on effectue la cyclisation à des températures allant de 0 à 150°C.

8. Procédé selon une ou plusieurs des revendications 1 à 7,
**caractérisé en ce qu'**
on prépare des composés de formule générale II à partir de dérivés de formule générale III 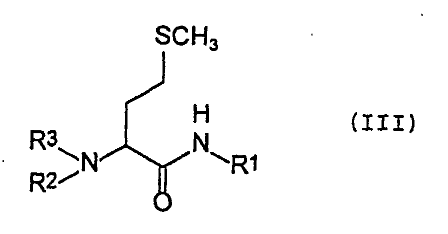 dans laquelle R¹, R², R³ ont la signification indiquée ci-dessus par réaction avec des acides halogènes carboxyliques de formule générale IV
X-Y-COOH (IV)
dans laquelle Y a la signification indiquée ci-dessus et X représente un halogène.

9. Procédé selon la revendication 8,
**caractérisé en ce qu'**
on conduit la réaction en présence d'une base.

10. Procédé selon la revendication 9,
**caractérisé en ce qu'**
on utilise comme base un hydroxyde de métal alcalin, de préférence l'hydroxyde de potassium.

11. Procédé selon une ou plusieurs des revendications 8 à 10,
**caractérisé en ce qu'**
on utilise comme acide halogène carboxylique de formule IV un acide alpha-halogène carboxylique, de préférence un acide alpha-halogène acétique.

12. Procédé selon la revendication 11,
**caractérisé en ce qu'**
on utilise l'acide bromacétique et l'acide chloracétique comme acide alpha-halogène carboxylique.

13. Procédé selon une ou plusieurs des revendications 8 à 12,
**caractérisé en ce qu'**
on conduit la réaction à des températures allant de -20°C à 150°C.

14. Procédé selon une ou plusieurs des revendications 8 à 13,
**caractérisé en ce qu'**
on conduit la réaction dans des solvants protiques polaires, de préférence le méthanol.

15. Procédé selon une ou plusieurs des revendications 1 à 14,
**caractérisé en ce qu'**
on cyclise le composé de formule générale II sans isolement préalable pour donner le composé de formule générale I.

16. Composés de formule générale II et leurs sels d'addition d'acides 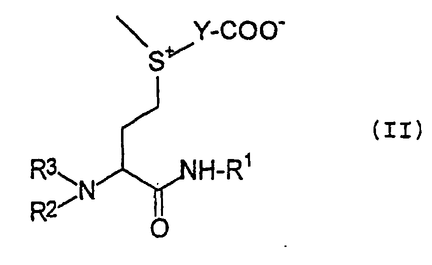 dans laquelle:
R¹ représente un alkyle en C₁ à C₈, un alcényle en C₂ à C₈, un alcynyle en C₂ à C₈, alcoxyalkyle en C₂ à C₈, un acyle en C₁ à C₈, qui le cas échéant peuvent se présenter sous forme linéaire ou ramifiée ainsi qu'être mono- ou polysubstitués avec les halogènes, des radicaux contenant des atomes de N, O, P, S, un cycloalkyle en C₃ à C₇; qui peut être saturé ou insaturé ainsi que mono- ou polysubstitué par des radicaux alkyle en C₁ à C₈, alcényle en C₂ à C₈, alcynyle en C₂ à C₈, acyle en C₁ à C₈, alcoxy en C₁ à C₈, alcoxyalkyle en C₂ à C₈ linéaire ou ramifié mono- ou polysubstitué par des radicaux contenant des halogènes, des atomes de N, O, P, S ou contenir dans le noyau des hétéroatomes tels que N, O, P, S, un aryle comme phényle ou naphtyle, un aralkyle comme benzyle ou phénéthyle, un hétéroaryle, comme furyle, pyrrolyle, pyridyle, un hétéroalkyle comme furfuryle, pyrrolylméthyle, piridineméthyle, furyléthyle, pyrrolyl éthyle, pyridyl éthyle, où les cycles mentionnés peuvent le cas échéant être mono- ou polysubstitués par des groupes alkyle en C₁ à C₈, alcényle en C₂ à C₈, alcynyle en C₂ à C₈, acyle en C₁ à C₈, alcoxy en C₁ à C₈, alcoxyalkyle en C₂ à C₈ linéaire ou ramifié, avec des halogènes, avec un nitrile, avec C(O)NH₂ ou des radicaux contenant N, O, P, S,
R² représente H, un alkyle en C₁ à C₈, un alcényle en C₂ à C₈, un alcynyle en C₂ à C₈, alcoxyalkyle en C₂ à C₈, qui peuvent se présenter éventuellement sous forme linéaire ou ramifiée ainsi qu'être mono- ou polysubstitués avec les halogènes, des radicaux contenant des atomes de N, O, P, S, un cycloalkyle en C₃ à C₇; qui peut être saturé ou insaturé ainsi que mono- ou polysubstitué par des radicaux alkyle en C₁ à C₈, alcényle en C₂ à C₈, alcynyle en C₂ à C₈, acyle en C₁ à C₈, alcoxy en C₁ à C₈, alcoxyalkyle en C₂ à C₈ linéaire ou ramifié mono- ou polysubstitué par des radicaux contenant des halogènes, des atomes de N, O, P, S et/ou contenir dans le noyau des hétéroatomes tels que N, O, P, S, un aryle comme phényle ou naphtyle, un aralkyle comme benzyle ou phénéthyle, un hétéroaryle, comme furyle, pyrrolyle, pyridyle, un hétéroalkyle comme furfuryle, pyrrolylméthyle, piridinméthyle, furyléthyle, pyrrolyl éthyle, pyridyl éthyle, où les cycles mentionnés peuvent le cas échéant être mono- ou polysubstitués par des groupes alkyle en C₁ à C₈, alcényle en C₂ à C₈, alcynyle en C₂ à C₈, acyle en C₁ à C₈, alcoxy en C₁ à C₈, alcoxyalkyle en C₂ à C₈ linéaire ou ramifié, avec des halogènes ou des radicaux contenant N, O, P, S,
R³ représente H, un acyle en C₁ à C₈, qui peut être le cas échéant linéaire ou ramifié, un groupe protecteur de peptide courant comme par exemple formyle, carbamoyle, benzyloxycarbonyle, tert-butoxycarbonyle, allyloxycarbonyle, trofluoroacétyle, et
Y représente un radical alkylène en C₁ à C₈ linéaire ou ramifié, qui le cas échéant peut être polysubstitué par un alkyle en C₁ à C₄, un halogène, un hydroxy ou un phényle, un arylalkyle en C₇ à C₁₅, qui le cas échéant peut être polysubstitué par un alkyle en C₁ à C₄, un halogène ou un hydroxy.

17. Composés de formule générale II selon la revendication 16, dans laquelle R² représente H, R³ un benzyloxycarbonyle et Y représente -CH₂-.

18. Composés de formule générale II selon la revendication 16, dans laquelle R² représente H, R³ représente un benzyloxycarbonyle, Y représente -CH₂- et R¹ représente un p-cyanophényle ou un p-carbamoyl phényle.

19. Utilisation des composés selon les revendications 16-18 pour la préparation d'intermédiaires pour substances bioactives.
